# NOUVEAU FASCICULE DE BREVET EUROPEEN

(11) **EP 1 432 390 B2**
(45) Date de publication et mention de la décision concernant l'opposition: **15.04.2015**
(45) Mention de la délivrance du brevet: 15.12.2010
(21) Numéro de dépôt: 02800154.3
(22) Date de dépôt: 24.09.2002
(51) Int. Cl.: A61Q 5/10, A61K 8/41, A61K 8/49

(54) **COMPOSITION DE TEINTURE A EFFET ECLAIRCISSANT POUR FIBRES KERATINIQUES HUMAINES**
FÄRBEMITTEL MIT AUFHELLER-EFFEKT FÜR MENSCHLICHE KERATINFASERN
DYEING COMPOSITION WITH A BRIGHTENING EFFECT FOR HAIR

(30) Priorité: 28.09.2001 FR 0112525
(43) Date de publication de la demande: 30.06.2004
(73) Titulaire: L'Oréal, 75008 Paris (FR)
(72) Inventeur: PASTORE, Florent, 92520 Rueil Malmaison (FR); GOURLAOUEN, Luc, 92600 Asnière (FR); LAGRANGE, Alain, 77700 Coupvray (FR)
(74) Mandataire: Wattremez, Catherine
(86) Numéro de dépôt international: PCT/FR2002/003252
(87) Numéro de publication internationale: WO 2003/028685

(56) Documents cités:
- EP-A- 0 370 470
- EP-A- 0 445 342
- EP-A1- 0 920 856
- EP-A2- 1 133 977
- WO-A-01/62759
- DE-A- 10 029 441
- DE-A- 19 926 377
- JP-A- H08 175 940
- US-A- 5 188 639
- US-A- 5 356 438
- E. A. HILL ET AL.: 'Ozone-Induced Chemiluminescence of Organic Analytes Deposited on Solid Substrates' ANALYTICAL CHEMISTRY vol. 54, no. 3, Mars 1982, pages 541 - 543
- J. KIM ET AL.: 'Phtophysical Properties of Hemicyanine Dyes Intercalated in Na-Fluorine Mica' J. PHYS. CHEM. A. vol. 104, no. 7, 2000, pages 1388 - 1392

## Description

L'invention concerne un procédé de teinture et d'éclaircissement des cheveux pigmentés ou colorés artificiellement qui présentent une hauteur de ton inférieur ou égale à 6, mettant en oeuvre une composition cosmétique comprenant au moins un colorant fluorescent dans la gamme des orangés soluble dans le milieu, en l'absence de colorants d'oxydation et d'agents oxydants.

Dans le domaine capillaire, Il existe principalement deux grands types de coloration capillaire.
Le premier est la coloration semi-permanente ou coloration directe qui fait appel à des colorants capables d'apporter à la coloration naturelle des cheveux, une modification plus ou moins marquée résistant à plusieurs shampooings. Ces colorants sont appelés colorants directs et peuvent être mis en oeuvre de deux manières différentes. Les colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors de coloration directe éclaircissante.
Le deuxième est la coloration permanente ou coloration d'oxydation. Celle-ci est réalisée avec des précurseurs de colorants dits "d'oxydation" qui sont des composés incolores ou faiblement colorés qui une fois mélangés à des produits oxydants, au moment de l'emploi, peuvent donner naissance par un processus de condensation oxydative à des composés colorés et colorants. Il est souvent nécessaire d'associer aux bases d'oxydation et coupleurs, un ou plusieurs colorants directs afin de neutraliser ou de rabattre les nuances trop en reflets rouges, orangés ou dorés, ou au contraire d'accentuer ces reflets rouges, orangés ou dorés.
Parmi les colorants directs disponibles, les colorants directs nitrés benzéniques ne sont pas suffisamment puissants, les indoamines, les colorants quinoniques ainsi que les colorants naturels présentent une faible affinité pour les fibres kératiniques et de ce fait conduisent à des colorations qui ne sont pas assez résistantes vis à vis des différents traitements que peuvent subir les fibres, et en particulier vis à vis des shampooings.

En outre, il existe un besoin d'obtenir un effet d'éclaircissement des fibres kératiniques humaines. Cet éclaircissement est obtenu classiquement par un procédé de décoloration des mélanines du cheveu par un système oxydant, généralement constitué par du peroxyde d'hydrogène associé ou non à des persels. Ce système de décoloration présente l'inconvénient de dégrader les fibres kératiniques et d'altérer leurs propriétés cosmétiques.

La demanderesse a donc recherché des composés permettant d'apporter des solutions aux problèmes évoqués ci-dessus, c'est à dire présentant une bonne affinité tinctoriale pour les fibres kératiniques, des bonnes propriétés de ténacités vis à vis des agents extérieurs, et en particulier vis à vis des shampooings, et qui permettent également d'obtenir un éclaircissement sans altération de la fibre.

C'est à la suite de ces recherches que la demanderesse a maintenant découvert de façon inattendue et surprenante que l'utilisation de colorants fluorescents dans la gamme des orangés permettait d'atteindre ces objectifs.

On connaît dans la littérature des compositions capillaires ou pour la peau comprenant des azurants optiques ou "optical brighteners" ou "fluorescent brighteners" en terminologie anglo-saxone, tels que décrits dans les brevets Canadien N°-1 255 603 ou les demandes de brevets WO-99/13845 et WO-00/71085. On connaît également des compositions de teinture des cheveux avec des composés permettant d'obtenir des nuances intenses comme celles décrites dans les demandes de brevet WO-01/62759, DE-100 29441 et DE-199 26377. Ces compositions n'éclaircissent cependant pas les cheveux ni la peau tout en les colorant.
Les demandes de brevets EP-0370470 et EP-0445342 décrivent des compositions cosmétiques comprenant un pigment insoluble qui peut être fluorescent (EP-0370470) formé par dissolution d'un colorant dans une résine. De telles compositions ne sont pas éclaircissantes.
Par ailleurs les brevets US-5 356 438 et US-5 188 639 décrivent des compositions pour colorer et conditionner les cheveux ou pour teindre et permanenter les cheveux; cependant ils n'ont pas pour objet d'éclaircir les cheveux en les colorant.

La présente invention a donc pour objet un procédé de teinture et d'éclaircissement des cheveux pigmentés ou colorés artificiellement qui présentent une hauteur de ton inférieur ou égale à 6, mettant en oeuvre une composition cosmétique comprenant, dans un milieu cosmétiquement acceptable, au moins un colorant fluorescent dans la gamme des orangés soluble dans le milieu, qui réémet la lumière qu'il absorbe dans la partie visible et en outre éventuellement ultra-violette du spectre, en lumière fluorescente de plus grande longueur d'onde dans le spectre du visible, en l'absence de colorants d'oxydation et d'agents oxydants, pendant un temps suffisant pour développer la coloration et l'éclaircissement désirés, après quoi on rince, on lave éventuellement au shampooing, on rince à nouveau et on sèche.

Par colorant fluorescent, on entend au sens de la présente invention un colorant qui, comme tout colorant classique, est une molécule qui colore par elle-même, est soluble dans le milieu cosmétique, absorbe la lumière du spectre visible et en outre éventuellement de l'ultra-violet (longueurs d'onde allant de 360 à 760 nanomètres) mais qui, contrairement au colorant classique, transforme l'énergie absorbée en lumière fluorescente de plus grande longueur d'onde émise dans la partie visible du spectre.

Un colorant fluorescent selon l'invention est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores, qui ne colorent pas, car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Selon la présente invention, on entend par cheveux pigmentés ou colorés artificiellement, des cheveux dont la hauteur de ton est inférieure ou égale à 6 (blond foncé) et de préférence inférieure ou égale à 4 (châtain).
L'éclaircissement des cheveux est évalué par la "hauteur de ton" qui caractérise le degré ou le niveau d'éclaircissement. La notion de « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Sciences des traitements capillaires » de Charles ZVIAK 1988, Ed.Masson, pp.215 et 278.
Les hauteurs de ton s'échelonnent de 1 (noir) à 10 (blond clair clair), une unité correspondant à un ton ; plus le chiffre est élevé et plus la nuance est claire.

Le colorant fluorescent selon l'invention est à différencier du pigment fluorescent. Le pigment est insoluble dans le milieu alors que le colorant est soluble dans le milieu cosmétique à température ambiante de l'ordre de 15 à 25°C. En outre, le colorant fluorescent selon l'invention est un colorant qui engendre une fluorescence sur le support cosmétique.

L'invention plus particulièrement a pour objet un procédé selon la revendication 1 mettant en oeuvre une composition cosmétique et caractérisée en outre par le fait qu'elle comprend, dans un milieu cosmétiquement acceptable, une quantité suffisante d'au moins un colorant fluorescent dans la gamme des orangés, pour qu'après application sur les cheveux, la composition donne une réflectance, mesurée entre 500 et 700 nm, qui est supérieure d'au moins 0,05%, et de préférence d'au moins 0,1%, à la réflectance des cheveux non traités.
Le procédé selon l'invention présente l'avantage d'éclaircir les cheveux sans les dégrader et en les colorant simultanément.

Selon la présente invention, le colorant fluorescent, éventuellement neutralisé, est soluble dans le milieu cosmétique à au moins 1 gramme par litre et de préférence à au moins 5 grammes par litre à la température de 25°C.

De préférence selon l'invention, le colorant fluorescent n'est pas un colorant de fluorescéine, ni un dérivé de xanthène tel que décrit dans la demande de brevet DE-199 26377, ni de cyclopentaquinoxalinium tel que décrit dans la demande de brevet DE-100 29441, ces composés n'étant pas dépourvus de toxicologie.

En outre, et de préférence, le procédé selon l'invention est susceptible d'éclaircir les cheveux dans une nuance qui, chiffrée dans le système C.I.E.L L*a*b* présente une variable b* supérieure ou égale à 6, avec un rapport b*/valeur absolue de a*, supérieur à 1,2 selon le test de sélection décrit ci-dessous.

### Test de sélection

La composition est appliquée sur des cheveux châtains à raison de 10 grammes de composition pour 1 gramme de cheveux châtains. La composition est étalée de façon à recouvrir l'ensemble des cheveux. On laisse la composition agir pendant 20 minutes à température ambiante (20 à 25°C). Les cheveux sont ensuite rincés à l'eau puis lavés avec un shampooing à base de lauryléther sulfate. Ils sont ensuite séchés. On mesure alors les caractéristiques spectrocolorimétriques des cheveux pour en déterminer les coordonnées L*a*b*.
Dans le système C.I.E.L L*a*b*, a* et b* indiquent deux axes de couleurs, a* indique l'axe de couleur vert/rouge (+a* est rouge, -a* est vert) et b* l'axe de couleur bleu/jaune (+b* est jaune et - b* est bleu) ; des valeurs proches de zéro pour a* et b* correspondent à des nuances grises.

Les colorants fluorescents utilisés dans le procédé selon la présente invention sont des colorants dans la gamme des orangés.

De préférence, la composition du procédé doit après application sur des cheveux, par exemple châtains, amener aux résultats suivants :
On s'intéresse aux performances de réflectance des cheveux lorsqu'on les irradie avec de la lumière visible dans la gamme de longueurs d'onde allant de 400 à 700 nanomètres.
On compare alors les courbes de réflectance en fonction de la longueur d'onde, des cheveux traités avec la composition de l'invention et des cheveux non traités. La courbe correspondant aux cheveux traités doit montrer une réflectance dans la gamme des longueurs d'onde allant de 500 à 700 nanoamètres supérieure à la courbe correspondant aux cheveux non traités.
Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités.
On entend par "supérieure", un écart d'au moins 0,05% de réflectance, et de préférence d'au moins 0,1 %. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités. De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Les colorants fluorescents selon la présente invention sont des composés connus et commercialisés.

On peut notamment citer parmi eux :
- le Photosensitiving Dye NK-557 commercialisé par la société UBICHEM qui présente la structure suivante : 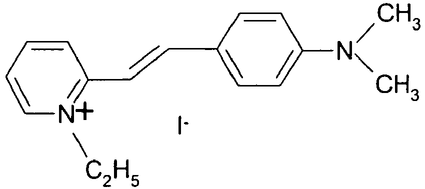 iodure de 2-[2-(4-diméthylamino)phényl éthényl]-1 éthyl-pyridinium.

Le ou les colorants fluorescents de la présente invention représentent de préférence de 0,01 à 20 %, plus préférentiellement de 0,05 à 10 % et plus particulièrement encore de 0,1 à 5% environ en poids du poids total de la composition.

Le milieu cosmétiquement acceptable est généralement constitué par de l'eau ou par un mélange d'eau et d'au moins un solvant organique.
A titre de solvant organique, on peut par exemple citer, les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions allant de préférence de 1 à 40 % en poids environ par rapport au poids total de la composition tinctoriale, et encore plus préférentiellement de 5 à 30 % en poids environ.

Le pH de la composition conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques humaines.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (I) suivante : 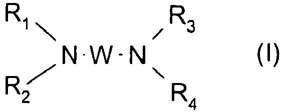 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ ou hydroxyalkyle en C₁-C₆.

La composition cosmétique mise en oeuvre dans le procédé conforme à l'invention peut, selon une forme de réalisation préférée, et en plus du ou des colorants fluorescents, comprendre un ou plusieurs colorants directs additionnels de nature non ionique, cationique ou anionique, qui peuvent par exemple être choisis parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine, et
- le 1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine, et
- le 1-amino-2-nitro-4-hydroxy-5-méthylbenzène.

La composition cosmétique mise en oeuvre dans le procédé conforme à l'invention peut également comprendre, en addition ou en remplacement de ces colorants benzéniques nitrés, un ou plusieurs colorants directs additionnels choisis parmi les colorants benzéniques nitrés jaunes, jaune-vert, bleus ou violets, les colorants azoïques, les colorants anthraquinoniques, naphtoquinoniques ou benzoquinoniques, les colorants indigoïdes, ou les colorants dérivés du triarylméthane.

Ces colorants directs additionnels peuvent notamment être des colorants basiques parmi lesquels on peut citer plus particulièrement les colorants connus dans le COLOR INDEX, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le COLOR INDEX, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP-A-0 714 954 et dont le contenu fait partie intégrante de la présente invention.

Parmi les colorants directs additionnels benzéniques nitrés jaunes et jaune-vert, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène, et
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule (II) suivante : 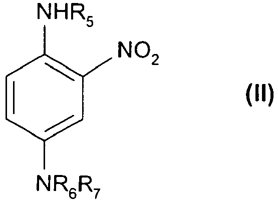 dans laquelle :
   - R₆ représente un radical alkyle en C₁-C₄, un radical β-hydroxyéthyle ou β-hydroxypropyle ou γ-hydroxypropyle ;
- R₅ et R₇, identiques ou différents, représentent un radical β-hydroxyéthyle, -β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français N°- 2 692 572.

Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

La composition cosmétique mise en oeuvre dans le procédé conforme à l'invention peut également comprendre divers adjuvants utilisés classiquement dans les compositions cosmétiques notamment de teinture des fibres kératiniques humaines, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des cations, des polymères cationiques ou amphotères, des silicones volatiles ou non volatiles, modifiées ou non modifiées, des agents filmogènes, des céramides, des agents conservateurs, des agents stabilisants, des agents opacifiants.

Parmi les agents épaississants, on préfère plus particulièrement utiliser les systèmes épaississants à base de polymères associatifs bien connus de l'homme de l'art et notamment de nature non ionique, anionique, cationique ou amphotère.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition cosmétique mise en oeuvre dans le procédé selon l'invention peut se présenter sous des formes diverses, telles que sous forme de liquides, de shampooings, de crèmes, de gels, ou sous toute autre forme appropriée.

Une forme particulièrement préférée selon la présente invention et qui constitue un autre objet de l'invention, est un shampooing colorant et éclaircissant comprenant dans un milieu aqueux cosmétiquement acceptable, au moins un colorant fluorescent tel que défini ci- dessus, et au moins un agent tensio-actif de préférence non ionique.
Dans ces shampooings, les agents tensio-actifs sont présents dans une proportion allant d'environ 4 à 30 % et de préférence d'environ 8 à 20% en poids par rapport au poids total de la composition de shampooing et les agents tensio-actifs non ioniques plus particulièrement préférés sont choisis parmi les alkylpolyglucosides.

Le temps nécessaire au développement de la coloration et à l'obtention de l'effet éclaircissant sur les cheveux est d'environ 5 à 60 minutes et plus particulièrement d'environ 5 à 40 minutes.
La température nécessaire au développement de la coloration et à l'obtention de l'effet éclaircissant sur les cheveux est généralement comprise entre la température ambiante (15 à 25°C) et 80°C et plus particulièrement entre 15 et 40 °C.

Les exemples qui suivent sont destinés à illustrer l'invention sans pour autant en limiter la portée.

### EXEMPLES 1 à 5

On a préparé les 3 compositions de teinture directe suivantes (teneurs en grammes) :

| **EXEMPLES** | **1** | **2**** | **3**** |
|---|---|---|---|
| Colorant fluorescent NK-557 | 0,5 | | - |
| Colorant fluorescent Jaune Brilliant B6GL | | 0,5 | |
| Colorant fluorescent Basic Yellow 2 | | | 0,5 |
| Hydroxyéthylcellulose | 1,6 | 1,6 | 1,6 |
| Alkyl (C8/C10 50/50) polyglucoside en solution aqueuse à 60% tamponnée | 6 MA* | 6 MA* | 6 MA* |
| Alcool benzylique | 8 | 8 | 8 |
| Polyéthylène glycol | 12 | 12 | 12 |
| Mélange de p-hydroxybenzoates de méthyle, butyle, éthyle, propyle, isobutyle | 0,12 | 0,12 | 0,12 |
| Eau déminéralisée q.s.p | 100 | 100 | 100 |

| | | | |
|---|---|---|---|
| MA* désigne Matière Active ** hors invention | | | |

On a également préparé 2 autres compositions pour comparaison, hors invention, ayant la même composition que les 3 compositions précédentes à la différence près du colorant qui est un colorant également orangé mais non fluorescent :

| **Exemples comparatifs hors invention** | **4** | **5** |
|---|---|---|
| Colorant non fluorescent ◆ | 0,5 | |
| Colorant non fluorescent ◆◆ | | 0,5 |
| Hydroxyéthyicellulose | 1,6 | 1,6 |
| Alkyl (C8/C10 50/50) polyglucoside en solution aqueuse à 60% tamponnée | 6 MA* | 6 MA* |
| Alcool benzylique | 8 | 8 |
| Polyéthylène glycol | 12 | 12 |
| Mélange de p-hydroxybenzoates de méthyle, butyle, éthyle, propyle, isobutyle | 0,12 | 0,12 |
| Eau déminéralisée q.s.p | 100 | 100 |

| | | |
|---|---|---|
| MA* désigne Matière Active | | |

### ◆ 2-nitro-4-hydroxy-1-amino-benzène

### ◆ ◆ Basic Orange 31 ou Vibracolor flame orange vendu par la société CIBA GEIGY de formule suivante :

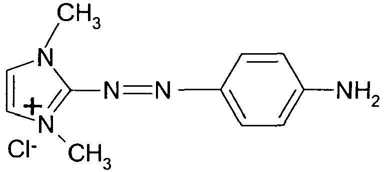

Chaque composition 1 à 5 a été appliquée, à raison de 10 g chacune, sur 1 g de mèches de cheveux naturels châtains et on a laissé pauser 20 minutes.
Les mèches ont ensuite été rincées à l'eau puis séchées.
Après 24 heures, les mèches ont été lues au Colorimètre Minolta CM 2002 dans le système L*a*b* et leurs hauteurs de ton ont été évaluées.

Les résultats ont été réunis dans le **tableau (I)** ci-après :

| | **HT*** | **L*** | **a*** | **b*** | **b* / a*** |
|---|---|---|---|---|---|
| Témoin non coloré | 4 | 24,24 | 3,82 | 4,51 | 1,18 |
| Composition 1 | **5** | 26,77 | 5,61 | **8,41** | **1,50** |
| Composition 2 | **4,5** | 25,85 | -1,07 | **8,92** | **8,34** |
| Composition 3 | **5** | 24,76 | 1,61 | **6** | **3,73** |
| Composition 4 | 4 | 23,99 | 6,43 | 5,71 | 0,89 |
| Composition 5 | 4 | 23,25 | 6,84 | 4,44 | 0,65 |

| | | | | | |
|---|---|---|---|---|---|
| HT* désigne Hauteur de Ton | | | | | |

D'après ces résultats, Il résulte clairement que la composition 1 colore en éclaircissant les cheveux.

Par ailleurs, les différences de réflectance entre le cheveu châtain non traité et le cheveu châtain traité par chacune des compositions 1 à 5 précédentes ont été les suivantes :

| **Composition** | **Longueur d'onde en nanomètres** | **Différence de réflectance en %** |
|---|---|---|
| **1** | 580 | 1,49 |
| **2** | 550 | 0,17 |
| **3** | 550 | 0,33 |
| **4** | 550 | -0,38 |
| **5** | 550 | -0,66 |

Les résultats sont reportés sur les courbes de réflectance du tableau (II) ci-après.

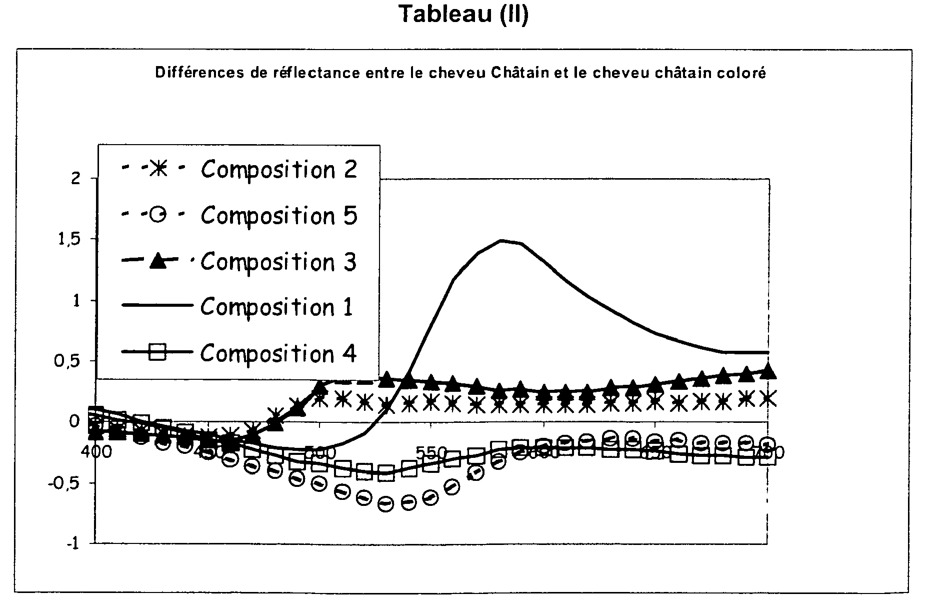

Ce graphique représente en abscisse la longueur d'onde de la lumière qui illumine les cheveux et en ordonnée la différence de réflectance des cheveux pour chaque longueur d'onde entre le cheveu châtain coloré et le cheveu châtain non coloré (hauteur de ton 4).

On remarque que pour les compositions 1, 2 & 3 (avec colorants fluorescents) la réflectance est positive (> 0%) sur presque la totalité du domaine de longueur d'onde alors que pour les compositions 4 & 5 (avec colorants non fluorescents) la réflectance est toujours négative et donc inférieure à la réflectance du cheveu châtain non coloré.

### Exemple 6

On a préparé le shampooing éclaircissant et colorant de composition suivante : *(teneurs exprimées en grammes de Matière Active MA***)*

| | |
|---|---|
| Tensio-actif non ionique : alkyl polyglucoside en solution aqueuse à 53% MA*(Plantacare 2000 UP vendu par COGNIS) | 15,9 MA* |
| 1,2-pentanediol | 0,1 |
| Glycérine | 7 |
| Acide anisique | 0,2 |
| Agent de conditionnement cationique: Quaternium 87 (methosulfate de dimethyl alkylamidoethylimidazolium en solution dans le propylène glycol ou Rewoquat W 575 PG vendu par GOLDSCHMIDT) | 0,15 MA* |
| Agent stabilisant : distéarate de polyéthylène glycol (PEG-50 distearate) | 4,2 |
| Chlorure de sodium | 3 |
| Acide citrique | 2,4 |
| Hydroxyde de sodium | 0,92 |
| Colorant fluorescent NK-557 | 0,5 |
| Eau déminéralisée q.s.p | 100 |

Puis on a réalisé 5 essais d'application de ce shampooing sur des mèches de cheveux châtains en appliquant le test de sélection décrit dans l'invention.
Les essais 3 et 4 ont été effectués pour montrer que les shampooings peuvent être superposés et conduire néanmoins à un résultat d'éclaircissement et de coloration simultanés.
Essai n°1 : une mèche de cheveux châtains de 1 gramme a été traitée avec 10 grammes de shampooing pendant 20 minutes à température ambiante. La mèche a été ensuite rincée à l'eau et séchée.
Essai n°2 : une mèche de cheveux châtains de 1 gramme a été traitée avec 0,4 gramme de shampooing pendant 1 minute à température ambiante. La mèche a été ensuite rincée à l'eau et séchée.
Essai n°3 : la mèche de cheveux de l'essai n°2 de 1 gramme a été à nouveau traitée avec 0,4 gramme de shampooing pendant 1 minute à température ambiante. La mèche a été ensuite rincée à l'eau et séchée.
Essai n°4 : la mèche de cheveux de l'essai n°3 de 1 gramme a été à nouveau traitée avec 0,4 gramme de shampooing pendant 1 minute à température ambiante. La mèche a été ensuite rincée à l'eau et séchée.

Les mèches ont été éclaircies et les mesures L*a*b* réunies dans le tableau (III) ci-dessous ont montré que la composition de l'exemple 6 entre dans les critères de sélection de la présente invention.

**Tableau (III)**

| | **L*** | **a*** | **b*** | **b*/valeur absolue de a*** | **Longueur d'onde en nanomètres** | **Différence de réflectance en%** |
|---|---|---|---|---|---|---|
| **Témoin**** | *22,34* | *3,14* | *3,96* | | | |
| Essai N°1 | *23,86* | *4,18* | *7,48* | *1,79* | *580* | *0,84* |
| Essai N°2 | *23,98* | *3,72* | *6,24* | *1,68* | *580* | *0,74* |
| Essai N°3 | *23,84* | *3, 91* | *6,15* | *1,57* | *580* | *0,74* |
| Essai N°4 | *24,33* | *4,26* | *7,13* | *1,67* | *580* | *1,04* |

| | | | | | | |
|---|---|---|---|---|---|---|
| ** *cheveu non traité* | | | | | | |

## Revendications

1. Procédé de teinture et d'éclaircissement des cheveux pigmentés ou colorés artificiellement qui présentent une hauteur de ton inférieur ou égale à 6, mettant en oeuvre une composition cosmétique comprenant, dans un milieu cosmétiquement acceptable, au moins un colorant fluorescent dans la gamme des orangés soluble dans le milieu, qui réémet la lumière qu'il absorbe dans la partie visible et en outre éventuellement ultra-violette du spectre, en lumière fluorescente de plus grande longueur d'onde dans le spectre du visible, en l'absence de colorants d'oxydation et d'agents oxydants, pendant un temps suffisant pour développer la coloration et l'éclaircissement désirés, après quoi on rince, on lave éventuellement au shampooing, on rince à nouveau et on sèche.

2. Procédé selon la revendication 1, **caractérisée par le fait que** les cheveux pigmentés ou colorés artificiellement présentent une hauteur inférieure ou égale à 4.

3. Procédé selon l'une quelconque des revendications précédentes, **caractérisée par le fait que** le colorant fluorescent est le colorant de structure suivante :

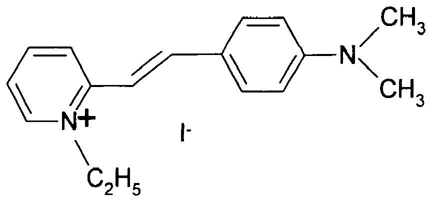

4. Procédé selon l'une quelconque des revendications précédentes, **caractérisée par le fait que** le ou les colorants fluorescents sont présents dans une concentration pondérale allant de 0,01 à 20% par rapport au poids total de la composition.

5. Procédé selon la revendication 4, **caractérisée par le fait que** le ou les colorants fluorescents sont présents dans une concentration pondérale allant de 0,05 à 10% par rapport au poids total de la composition.

6. Procédé selon la revendication 5, **caractérisée par le fait que** le ou les colorants fluorescents sont présents dans une concentration pondérale allant de 0,1 à 5% par rapport au poids total de la composition.

7. Procédé selon l'une quelconque des revendications précédentes, **caractérisée par le fait que** le milieu cosmétiquement acceptable est constitué par de l'eau ou par un mélange d'eau et d'au moins un solvant organique.

8. Procédé selon l'une quelconque des revendications précédentes, **caractérisée par le fait qu'**elle présente un pH allant de 3 à 12, et de préférence de 5 à 11.

9. Procédé selon l'une quelconque des revendications précédentes, **caractérisée par le fait qu'**elle comprend en outre au moins un colorant direct additionnel de nature non ionique, cationique ou anionique.

10. Procédé selon la revendication 9, **caractérisée par le fait que** les colorants directs additionnels sont choisis parmi les colorants benzéniques nitrés.

11. Procédé selon la revendication 9, **caractérisée par le fait que** les colorants directs additionnels sont choisis parmi les colorants azoïques, les colorants anthraquinoniques, naphtoquinoniques ou benzoquinoniques, les colorants indigoïdes, ou les colorants dérivés du triarylméthane.

12. Procédé selon l'une quelconque des revendications 9 à 11, **caractérisée par le fait que** le ou les colorants directs additionnels représentent de 0,0005 à 12 % en poids du poids total de la composition.

13. Procédé selon la revendication 12, **caractérisée par le fait que** le ou les colorants directs additionnels représentent de 0,005 à 6 % en poids du poids total de la composition.

## Patentansprüche

1. Verfahren zum Färben und Aufhellen von künstlich gefärbten oder pigmentierten Haaren mit einer Tontiefe kleiner gleich 6 unter Verwendung einer kosmetischen Zusammensetzung, die in einem kosmetisch unbedenklichen Medium mindestens einen in dem Medium löslichen Fluoreszenzfarbstoff im Orangebereich, der das von ihm im sichtbaren Teil und außerdem gegebenenfalls im ultravioletten Teil des Spektrums absorbierte Licht als Fluoreszenzlicht größerer Wellenlänge im sichtbaren Spektrum wieder abstrahlt, in Abwesenheit von Oxidationsfärbemitteln und Oxidationsmitteln umfasst, über einen zur Entwicklung der gewünschten Färbung und der gewünschten Aufhellung ausreichenden Zeitraum, wonach man spült, gegebenenfalls mit Shampoo wäscht, erneut spült und trocknet.

2. Verfahren nach Anspruch 1, **dadurch gekennzeichnet, dass** die künstlich gefärbten oder pigmentierten Haare eine Tontiefe kleiner gleich 4 aufweisen.

3. Verfahren nach einem der vorhergehenden Ansprüche, **dadurch gekennzeichnet, dass** es sich bei dem Fluoreszenzfarbstoff um den Farbstoff der folgenden Struktur handelt:

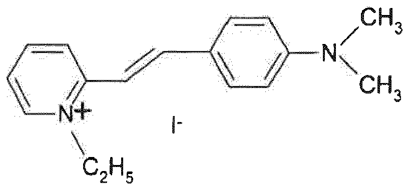

4. Verfahren nach einem der vorhergehenden Ansprüche, **dadurch gekennzeichnet, dass** der Fluoreszenzfarbstoff bzw. die Fluoreszenzfarbstoffe in einer Konzentration von 0,01 bis 20 Ges.-%, bezogen auf das Gesamtgewicht der Zusammensetzung, vorliegt bzw. vorliegen.

5. Verfahren nach Anspruch 4, **dadurch gekennzeichnet, dass** der Fluoreszenzfarbstoff bzw. die Fluoreszenzfarbstoffe in einer Konzentration von 0,05 bis 10 Gew.-%, bezogen auf das Gesamtgewicht der Zusammensetzung, vorliegt bzw. vorliegen.

6. Verfahren nach Anspruch 5, **dadurch gekennzeichnet, dass** der Fluoreszenzfarbstoff bzw. die Fluoreszenzfarbstoffe in einer Konzentration von 0,1 bis 5 Gew.-%, bezogen auf das Gesamtgewicht der Zusammensetzung, vorliegt bzw. vorliegen.

7. Verfahren nach einem der vorhergehenden Ansprüche, **dadurch gekennzeichnet, dass** das kosmetisch unbedenkliche Medium aus Wasser oder einer Mischung von Wasser und mindestens einem organischen Lösungsmittel besteht.

8. Verfahren nach einem der vorhergehenden Ansprüche, **dadurch gekennzeichnet, dass** die Zusammensetzung einen pH-Wert von 3 bis 12 und vorzugsweise von 5 bis 11 aufweist.

9. Verfahren nach einem der vorhergehenden Ansprüche, **dadurch gekennzeichnet, dass** die Zusammensetzung außerdem mindestens einen zusätzlichen Direktfarbstoff nichtionischer, kationischer oder anionischer Natur umfasst.

10. Verfahren nach Anspruch 9, **dadurch gekennzeichnet, dass** die zusätzlichen Direktfarbstoffe aus Nitrobenzol-Farbstoffen ausgewählt sind.

11. Verfahren nach Anspruch 9, **dadurch gekennzeichnet, dass** die zusätzlichen Direktfarbstoffe aus AzoFarbstoffen, Anthrachinon-, Naphthochinon- oder Benzochinon-Farbstoffen, Indigoid-Farbstoffen oder von Triarylmethan abgeleiteten Farbstoffen ausgewählt sind.

12. Verfahren nach einem der Ansprüche 9 bis 11, **dadurch gekennzeichnet, dass** der zusätzliche Direktfarbstoff bzw. die zusätzlichen Direktfarbstoffe 0,0005 bis 12 Gew.-%, bezogen auf das Gesamtgewicht der Zusammensetzung, ausmacht bzw. ausmachen.

13. Verfahren nach Anspruch 12, **dadurch gekennzeichnet, dass** der zusätzliche Direktfarbstoff bzw. die zusätzlichen Direktfarbstoffe 0,005 bis 6 Gew.-%, bezogen auf das Gesamtgewicht der Zusammensetzung, ausmacht bzw. ausmachen.

## Claims

1. Method for dyeing and brightening artificially coloured or pigmented hair which has a tone level less than or equal to 6, said method employing a cosmetic composition comprising, in a cosmetically acceptable medium, at least one fluorescent dye from the range of oranges which is soluble in the medium and which re-emits the light which it absorbs in the visible and also optionally in the ultraviolet part of the spectrum as fluorescent light with a longer wavelength in the visible spectrum, in the absence of oxidation dyes and oxidizing agents, for a time sufficient to develop the desired colouring and brightening, after which rinsing, optional washing with a shampoo, rinsing again and drying are carried out.

2. Method according to Claim 1, **characterized in that** the artificially coloured or pigmented hair has a tone level less than or equal to 4.

3. Method according to either one of the preceding claims, **characterized in that** the fluorescent dye is the dye having the following structure:

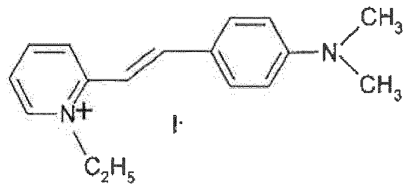

4. Method according to any one of the preceding claims, **characterized in that** the fluorescent dye(s) are present in a concentration by weight ranging from 0.01 to 20% relative to the total weight of the composition.

5. Method according to Claim 4, **characterized in that** the fluorescent dye(s) are present in a concentration by weight ranging from 0.05 to 10% relative to the total weight of the composition.

6. Method according to Claim 5, **characterized in that** the fluorescent dye(s) are present in a concentration by weight ranging from 0.1 to 5% relative to the total weight of the composition.

7. Method according to any one of the preceding claims, **characterized in that** the cosmetically acceptable medium consists of water or of a mixture of water and of at least one organic solvent.

8. Method according to any one of the preceding claims, **characterized in that** the composition has a pH ranging from 3 to 12 and preferably from 5 to 11.

9. Method according to any one of the preceding claims, **characterized in that** the composition also comprises at least one additional nonionic, cationic or anionic direct dye.

10. Method according to Claim 9, **characterized in that** the additional direct dyes are chosen from nitrobenzene dyes.

11. Method according to Claim 9, **characterized in that** the additional direct dyes are chosen from azo dyes, anthraquinone, naphthoquinone or benzoquinone dyes, indigoid dyes or triarylmethane-derived dyes.

12. Method according to any one of Claims 9 to 11, **characterized in that** the additional direct dye(s) represent from 0.0005 to 12% by weight of the total weight of the composition.

13. Method according to Claim 12, **characterized in that** the additional direct dye(s) represent from 0.005 to 6% by weight of the total weight of the composition.
